(19) Europäisches Patentamt
European Patent Office
Office européen des brevets

(11) Publication number: **0 429 644 B1**

(12) **EUROPEAN PATENT SPECIFICATION**

(45) Date of publication of patent specification: **20.09.95**

(51) Int. Cl.$^6$: **A61K 51/00**, A61K 49/00, A61K 39/44, C07K 17/06, C07F 5/00, C07F 13/00, C07C 331/28

(21) Application number: **90914273.9**

(22) Date of filing: **23.05.90**

(86) International application number: **PCT/US90/02910**

(87) International publication number: **WO 90/14881 (13.12.90 90/28)**

Divisional application 95200465.3 filed on 23/05/90.

(54) **CHELATING AGENTS FOR ATTACHING METAL IONS TO PROTEINS.**

(30) Priority: **26.05.89 US 358917**

(43) Date of publication of application: **05.06.91 Bulletin 91/23**

(45) Publication of the grant of the patent: **20.09.95 Bulletin 95/38**

(84) Designated Contracting States: **AT BE CH DE DK ES FR GB IT LI NL SE**

(56) References cited:
**WO-A-84/03698**
**WO-A-86/06605**
**WO-A-88/01178**

**INORGANIC CHEMISTRY, vol. 25, no. 16, 1986; M.W. BRECHBIEL et al., pp. 2772-2781**

(73) Proprietor: **Akzo Nobel N.V.**
**Velperweg 76**
**NL-6824 BM Arnhem (NL)**

Proprietor: **Subramanian, Ramaswamy**
**352 Catoctin Avenue**
**Frederick, MD 21701 (US)**

(72) Inventor: **SUBRAMANIAN, Ramaswamy**
**352 Catoctin Avenue**
**Frederick, MD 21701 (US)**
Inventor: **COLONY, James L.**
**704 North 75th Street**
**SEATTLE; WA 98103 (US)**

(74) Representative: **Hermans, Franciscus G.M. et al**
**Patent Department**
**AKZO NOBEL N.V.**
**Pharma Division**
**P.O. Box 20**
**NL-5340 BH Oss (NL)**

**EP 0 429 644 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to new chelating agents for attaching metal ions to proteins such as albumin, transferrin, antibodies and etc.

### BACKGROUND OF THE INVENTION

Attachment of metal ions to proteins leads to several useful products. These include fluorescent, radioactive and paramagnetic metal ions attached proteins that can be used as probes in vivo in biological systems and in vitro in analytical systems such as radioimmunoassays. For example, attachment of radionuclides to monoclonal antibodies that recognize tumor associated antigens provides radioimmunoconjugates useful for cancer diagnosis and therapy. The monoclonal antibodies are used as carriers of desired substances to specific sites in vivo. Several chelating agents such as diethylenetriaminepentaacetic acid (DTPA), ethylene-diaminetetraacetic acid (EDTA) and macrocyclics have been reported to form stable complexes when attached to protein. In particular, M.W. Brechbiel et al. (Inorg. Chem. 25 (16), 2772-81, 1986) discloses 1-(p-isothiocyanatobenzyl) derivatives of DTPA and EDTA and also a protein linked diethylenetriaminetetraacetic acid (DTTA). However, kinetic instability of the radioimmunoconjugate or the chelate under physiological conditions results in the breakdown of these complexes. Despite several attempts to modify the mode of binding, structure of chelate and etc., in vivo administration of such radioimmunoconjugates results in accumulation of radioactivity in non-target tissues, particularly the liver. Hence, there is an obvious need for new chelating agents for binding radiometals to antibodies forming complexes that do not disassociate when administered to a patient.

### SUMMARY OF THE INVENTION

It is an object of this invention to provide a new set of chelating agents for attaching metal ions to proteins and thereby provide an aqueous solution containing antibodychelate conjugate that is stable in vivo.

It is further an object of this invention to provide a set of chelating agents to bind a variety of metal ions, including In, Y, Gd, Tb, Cu, Co, Sm, Rh, Ru, Re, Tc, Fe, Pb, Ba, actinides, lanthanides and transition metal ions.

It is further an object of this invention to synthesize new chelation structures useful for attaching metal ions to proteins, including monoclonal antibodies.

It is another object of this invention to obtain versatile chelating agents that are not only suitable for binding to low molecular weight proteins such as albumin and IgG, but also to high molecular weight proteins such as IgM ($9x10^5$), lipoproteins ($2x10^6$) and etc.

It is still another object of this invention to obtain an improved method for preparing metal chelate conjugated antibodies.

An additional object of this invention is to obtain chelation structures that provide a high metal ion concentration per antibody molecule without destroying the biological activity of the conjugated protein to a significant extent.

It is still another object of this invention to obtain fluorescent labeled proteins by attaching fluorescent/luminescent metal ions to protein-chelate conjugates.

It is further the object of this invention to obtain metal ion binding reagents that can be attached to chromatographic column materials such as polymers and gels, forming chelate affinity columns.

These and other objects are accomplished by one or more of the embodiments of the present invention.

### BRIEF DESCRIPTION OF THE DRAWINGS

The bifunctional chelating agents of the invention are shown in Figure 1. In Figure 1, "a" is LiLo1, and "b" is LiLo2.

Figure 2 illustrates synthetic procedures for obtaining the metal-binding chelating agent LiLo.

Figure 3 compares the stabilities of Indium-111 labeled HSA-LiLo1 and HSA-DTPA in phosphate buffered saline (PBS), (0.05M, pH 7.2) at 37°C.

Figure 4 compares the biological activity (immunoreactivity) of a protein-chelate conjugate (LiLo1 conjugated to IgM isotype human monoclonal antibody 16-88) to that of the unconjugated protein (16-88).

Figure 5 illustrates the purification of $(16.88)LiLo^{111}In$ by gel filtration chromatography.

DESCRIPTION OF PREFERRED EMBODIMENTS

One embodiment of this invention is directed to a buffered solution containing an antibody or protein chelate conjugate wherein all the metal ions are bound to protein via the chelator.

Attachment of radiometals to antibodies requires first the attachment of ligands to antibodies. Ligands, also termed chelators or chelating agents, are compounds that can bind radiometals efficiently through the formation of coordinate bonds. Ligands such as polyaminocarboxylic acids, macrocyclics, crown ethers, cryptands and cyclams can be used to bind radiometals. The choice of the ligand depends on the choice of the radionuclide involved and the properties of the protein conjugated. For radioimmunoimaging and therapy the radionuclides of interest include $^{111}$In, $^{90}$Y, $^{203}$Pb, $^{155}$Sm, $^{101}$Rh, $^{97}$Ru, $^{67}$Cu, $^{201}$Tl, $^{99}$mTc, $^{55}$Co $^{186}$Re, $^{188}$Re, $^{212}$Bi and $^{213}$Bi. For paramagnetic metal ions, Gd and other similar metal ions can be used. For fluorescence or luminescence based investigations, metal ions such as Tb, Eu, Ru and Rh can be employed.

Ligands suitable for attachment to both proteins and metal ions are often called bifunctional chelating agents. In addition to having a metal binding moiety, these compounds also possess reactive functional groups useful for attachment to proteins. The reactive functional groups are already known in the art. Examples of these groups are isothiocyanato, bromoacetamido, diazo, N-hydroxysuccinimide esters and anhydrides. These groups can be incorporated into chelating agents. The metal ions con be part of a linker molecule containing several carbon atoms [$(CH_2)n$] and several other groups. Some of the factors to be considered while constructing an immunoconjugate are: (1) They must be stable, (2) readily bind the metal ions and (3) retain the biological activity of the protein. The chelation structures described in this invention satisfy these conditions.

In addition to the chelating agents, this invention comprises the synthesis of a family of bifunctional chelating agents starting from (p)-nitrobenzyl bromide and diethylmalonate. From the condensed products, several amines such as (p)$NO_2$-Ph-$CH_2$-$CH(CH_2NH_2)_2$, (p)$NO_2$-Ph- $CH(CH_2.CH_2.\ NH_2)_2$ and

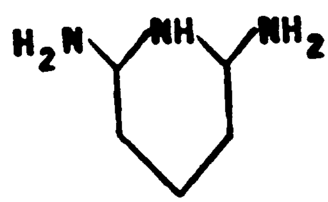

with (p)$NO_2$-Ph-$CH_2$ at C-3 can be prepared. They are the key intermediates from which the metal binding chelating agents a and b in Figure 1 can be synthesized. These reagents also possess a reactive functional group such as -NCS, -NH-CO-$CH_2$Br, or -$N_2$Br, which can be used to attach them to proteins.

Conventional chelating agents lead to several different products when coupled with proteins (e.g. aggregates). Often use of linkers such as dianhydrides may be the reason for this. By employing linkers such as isothiocyanate or -NH-CO-$CH_2$Br, these complications are avoided.

For preparing the chelates of this invention all reactions were carried out in non-aqueous solvents, such as methanol methylene chloride and THF, and purification was performed by silica gel chromatography. There are several significant aspects to this approach. Using the same sequence of reactions, we have obtained chelates containing carboxyl groups, and/or phenoxyl groups, macrocyclic chelating agents, reagents containing several metal binding groups, and reagents containing a conformationally rigid moiety. These reagents offer a wide variety of metal chelates with unique and useful properties. Further, the sequence of reactions defined can be employed to obtain several other bifunctional reagents, as well as polymeric starburst ligands.

Using the above mentioned sequence of reactions, one can obtain a series of reagents useful for attaching different types of metal ions to proteins, e.g. LiLo for $^{111}$In/$^{90}$Y.

These reagents can be coupled to proteins by gentle and simple means. One procedure involves incubating the protein and chelating agents in a suitable buffer at pH 6-9 for 1-3 hrs at 37 °C., and purifying the protein-chelate conjugate formed by gel filtration chromatography. We coupled these reagents to human serum albumin and the resultant chelate conjugated albumin binds metals such as $^{57}$Co with very high efficiency(>95%). The proteins can be monoclonal or polyclonal antibodies, or other proteins such as transferrin, albumin and derivatives thereof. One can modify the linkers in order to obtain maximum immunoreactivity. The metal ion can be transition metal ions, paramagnetic, radioactive and fluorescent/ luminescent metal ions, lanthanides, actinides and others. These include but are not limited to Gd, Tb, Eu,

Ru, Rh, Pd, Pb, Sm, Tb, Ga, In, Tl, Fe, Co, Ni, Re, Y, Tc, Cr, Os, Ir, Sb and Cu. The procedure for attaching the metal ions involves incubation of the immunoconjugate with a metal salt solution at pH 5-9, at temperatures of 20-37°C. for a period of 0.5-6 hrs., or at 4°C. for 24 hours, followed by gel filtration chromatography.

If the metal ion involved is not radioactive, the metal-chelate conjugate can be prepared and purified first, and then attached to the protein to obtain metal-chelateprotein conjugate. Because of the presence of polyaminocarboxylates in the case of LiLo one can easily achieve high metal ion concentration. In radioimmunotherapy and magnetic resonance imaging this is of paramount importance, as often the antibody uptake by tumor in patients is <0.005% I.D/g.

All reagents a and b in Figure 1 are polyaminocarboxylates that will bind radiometals and other metal ions, and can be attached to proteins such as monoclonal antibodies. The choice of the reagent depends on

a. the application (or the end use)
b. the nature of metal ion
c. the protein (biological properties).

For example, when LiLo1 was attached to HSA and labeled with Indium-111, and the rate of decomposition of the radioimmunoconjugate was determined at 37°C. by a DTPA chase technique, it was found that HSA-LiLo1-$^{111}$In loses $^{111}$In at the rate of <1%/day. Under similar experimental conditions, HSA-DTPA-$^{111}$In loses $^{111}$In at the rate of 10% per day. These results show that LiLo may be a better chelator for Indium than DTPA.

The presence of unbound metal ions, if any, in the purified conjugate can be determined using standard analytical techniques such as thin layer chromatography (ascending), NMR, IR, or visible or fluorescence spectroscopy.

Example I

Conjugation and Stability Studies:

For stability studies, HSA-LiLo conjugate was first prepared by reacting HSA (30 mg/ml, 0.05M PBS solution, pH7.2) with Lilo (HSA: LiLo molar ratio 1:10) at 37°C. for 2 hours. Unbound chelate was removed by gel filtration chromatography (G-50 Sephadex, Pharmacia). The conjugate (HSA-Lilo,) was collected and the concentration of the protein determined by absorption at 280 nm. The preparation of HSA-DTPA involved the addition of DTPA dianhydride to HSA in PBS buffer (0.05m, pH7.2) and incubation at room temperature for 15 min. Unbound chelate was removed by gel filtration chromatography as described above.

The conjugate was labeled with In-111 in a buffer (0.05M citrate/0.5 M acetate, pH 5.5) DTPA was added to bind any unbound Indium-111, and the labelled conjugate was purified by G-50 or G-25 gel filtration chromatography. The purity of the immunoconjugate was determined by ITLC in PBS buffer using Gelman ITLC-SG chromatography paper.

Stability studies were carried out in 0.05M PBS buffer pH 7.2 at 37°C. At periodic intervals an aliquot of the solution was removed and treated with excess DTPA solution to chelate any dissociated $^{111}$In. Thin layer chromatography analysis using ITLC-SG strip (Gelman Sciences) in PBS buffer (0.05M, pH 7.2) was carried out, and the ITLC strip was cut into two pieces, and the indium (111) activity was determined using a gamma counter.

$$100 \times \frac{\text{radioactivity at the bottom strip}}{\text{radioactivity at the (bottom + top) strips}}$$

equals the percentage of $^{111}$In bound to protein.

Results obtained with $^{111}$In-HSA-LiLo and $^{111}$In-HSA-DTPA are shown in Figure 3.

The chelator LiLo was also conjugated to a human monoclonal antibody 16.88. Human monoclonal antibody 16.88 is an IgM isotype produced by a spontaneously transformed human lymphoblastoid cell line, obtained from the peripheral blood lymphocytes of colon carcinoma patients, which expresses the Epstein - Barr nuclear antigen. Attaching radiometals to IgM monoclonals has been reported to be difficult when compared with IgG's or other proteins. Conventional chelating agents such as DTPA anhydrides lead to aggregation of the antibody; in addition, the recovery of the radiolabeled MoAb is low. Such a low recovery was not observed in the case of radiolabeled 1688-Lilo.

The new chelating agent Lilo was conjugated to 16.88. By a competitive binding assay, the immunoactivity of these conjugates in comparison with unconjugated 16.88 was determined. In this assay antigen was coated on a microtiter plate and treated with 16.88 labeled with radioiodine. This was then allowed to complete with unconjugated 16.88 or 16.88 - Lilo. Results obtained by this assay show that 16.88 LiLo was as immunoreactive as unconjugated 16.88. Results obtained with 16-88 are shown in Figure 4.

The number of chelates per MoAb was determined by known methods in the state of the art. In general, this method involved the addition of known amounts of $InCl_3$ containing $^{111}InCl_3$ to the immunoconjugate, and incubating the mixture in 0.05M citrate/0.5M acetate buffer, pH 5.5, for 30 min. at room temperature (23 - 27 °C.) This was followed by the addition of excess DTPA solution to chelate the unbound Indium. After 5 minutes, an aliquot of the mixture was spotted on TLC (thin layer chromatography). Chromatography was carried out using 10% ammonium acetate (aqueous) and methanol (1:1 by volume) as the eluant mixture. $^{111}$In labeled protein stays at the origin whereas In-DTPA moves (Rf = 0.75). By cutting and counting the various portions of TLC, one could determine the number of chelates per protein, since the concentrations of protein and Indium taken in the reaction mixture are known. Normally this ratio was 1-5.

The immunoreactivity of these conjugates was also determined by a direct cell binding assay as suggested by Lindmo, T. et al. J. Immunol.Meth, 72(1), 77-79(1984). In this assay 16.88 - chelate conjugates were labeled with $^{111}$In and purified by gel filtration chromatography. Figure 5 describes the purification of (16.88)LiLo$^{111}$In by gel filtration (Sephadex, G-25) chromatography. The first peak (fractions #3-#6, 0.9ml/fraction) contains the radioimmuno conjugates. PBS buffer (0.05M, pH 72) was used as the eluant. The labeled conjugates were incubated with either WiDr cell lines or the antigen, and the percentage of activity bound to the cells or antigen was determined. The immunoreactivity of 16.88 - LiLo-$^{111}$In was >95%.

These results confirm that the chelator LiLo can be used to attach $^{111}$In to the human monoclonal antibody 16.88 with no significant loss of immunoactivity.

As observed in the case of HSA-chelator conjugates, 16.88 - Lilo - $^{111}$In was stable in PBS buffer solution (<1% per day) under physiological conditions. For $^{111}$In and $^{90}$Y, which has properties similar to that of indium, 16.88 - LiLo will be a good conjugate for in vivo applications.

Example II

The synthesis of LiLo is described in Figure 2. P-nitrobenzylbromide was condensed with diethyl malonate to obtain p-nitrobenzyl diethylmalonate.

p-nitrobenzyl diethylmalonate was directly reduced to a diamine, and condensed with DTPA to obtain LiLo 1. This condensation was carried out in three different ways.

1. Condensation between the diamine and DTPA methyl ester.
2. Coupling between the diamine and DTPA cyclic anhydride.
3. A reaction between the diamine and DTPA acid by the mixed anhydride method.

In our experiments with paths 2 and 3, the product obtained was converted to a methyl ester, purified by silica gel chromatography and converted to LiLo 1. Reduction of dinitrile (III) to a diamine, followed by carboxymethylation, reduction with hydrogen in the presence of Palladium on activated carbon, and treatment with $CSCl_2$ and trifluoroacetic acid yielded LiLo2.

Details of the synthesis are give below:

A. 2(4-nitrobenzyl)diethylmalonate(I):

6.9g (0.3m) of sodium metal was added gradually to a stirred solution of 300ml absolute ethanol under a nitrogen atmosphere. After all metal had dissolved 96g (0.6m) diethylmalonate was slowly added dropwise to the solution. This was followed by the addition of 65g (0.3m) of 4-nitrobenzylbromide in portions over one hour. The solution was heated to reflux for 24hrs., and precipitated byproduct was filtered off. The solution was evaporated and the crystallized product was filtered and washed with ethanol. Yield 34.9g(40%)-,m.pt.60 °C. Ir 1736cm$^{-1}$, 1524cm$^{-1}$, 1346cm$^{-1}$, 1151cm$^{-1}$, 852cm$^{-1}$, KBr pellet. NMR, CDCl3, 8.14d, 2H; 7.37d, 2H; 4.15, 7, 4H; 3.64,t 1H; 3.30,d.2H; 1.22,t,6H.

B. 2-(4-nitrobenzyl)-1,3-propanediol (II):

11.3g (0.038m) (I) was dissolved in 20ml anhydrous THF and injected into a stirred solution of 190 ml 1M $BH_3$-THF solution under a nitrogen atmosphere at 4 °C. The solution was slowly heated to reflux for 18

hours. The solution was then cooled to room temperature and methanol was added in small portions to quench the excess $BH_3$ complex. The solution was then rotoevaporated. Further purification was performed by silica gel chromatography. Yield was 6.3g (78%), mpt. 85 ° C. IR(KBr pellet) 3260 $cm^{-1}$, 1539 $cm^{-1}$, 1351 $cm^{-1}$, 1030 $cm^{-1}$. NMR 3.73ppm, d,4H; 2.78ppm, d, 2H; 2.35ppm,s,2H; 2.03ppm,s,1H.

C. 3-(4-nitrobenzyl)1,5-pentanedinitrile(III):

6.9g(0.036m)p-toluenesulfonylchloride (tosyl) was dissolved in 10ml dry pyridine and cooled to 4 ° C. 2.5g (0.012m) II was diluted in 5ml dry pyridine and added slowly to the stirred tosyl solution with cooling. The solution was stirred for 15 min. at 4 ° C., then allowed to warm to room temperature and stirred for 3 hrs. The reaction was quenched by pouring the solution slowly with stirring into 100ml of 30% HCl at 4 ° C. The product was extracted with $CH_2Cl_2$, evaporated and crystallized in ethanol to produce the tosyl derivative. mpt 85-89C. IR(KBr pellet) 1524 $cm^{-1}$, 1352 $cm^{-1}$, 1194 $cm^{-1}$, 1176 $cm^{-1}$.

3.1g KCN was suspended in a stirred solution of 30 ml absolute ethanol at 4 ° C. The above tosyl derivative was dissolved in a 1:1 mixture of $CH_2Cl_2$:EtOH and added to the KCN mixture. The solution was slowly heated to reflux for 24 hours. The solution was then rotoevaporated, extracted with $CH_2Cl_2$, filtered and evaporated. The oil was then run down a silica gel column to be isolated as an orange oil (III). 1.9g Yield 70%, IR (NaCl plate) 2248 $cm^{-1}$, NMR 2.96ppm,s,2H; 2.5ppm,s,4H; 2.13ppm,m,1H.

EXAMPLE III

Synthesis of LiLo1:

A. 3-(4-nitrobenzyl)diamidomalonate(VII):

4.3g (I) was dissolved in 150 ml of methanol, cooled to 4 ° C., and ammonia was bubbled through the solution to the point of saturation. The solution was stoppered and kept at 4 ° C. for 48 hrs. The precipitated product was filtered and rinsed with methanol. IR (KBr pellet) 3441 $cm^{-1}$, 3392 $cm^{-1}$, 1678 $cm^{-1}$, 1657 $cm^{-1}$.

B. 2-(4-nitrobenzyl)1,3-diaminopropane (VIII):

Dihydrochloride Salt

2.0g (VII) was suspended in THF (tetrahydrofuran) (20ml) and added to a stirred 60ml 1m BH3.THF solution at 4 ° C. The reaction was slowly heated to reflux for 15 hrs. The solution was quenched with methanol and HCl bubbled through with no external cooling until product separated as a yellow oil. The solid was filtered and rinsed with methanol. Yield 2.17g (91%), IR (KBr pellet) 2934 $cm^{-1}$, 1600 $cm^{-1}$, 1514 $cm^{-1}$ 1353 $cm^{-1}$.

C. Nitro "LiLo" Methyl Ester (IX):

300mg (VIII) and 3.3g DTPA pentamethyl ester were dissolved in 10ml dry methanol and heated to reflux for 7 days. The reaction mixture was evaporated and purified by silica gel column chromatography. The product was fluorescamine negative. Yield 17.5%. IR (KBr pellet) 1741 $cm^{-1}$, 1654 $cm^{-1}$, 1348 $cm^{-1}$, 1204 $cm^{-1}$.

D. ICN-Lilo-Methyl ester(X):

15 mg (IX) was dissolved in 5 ml methanol and cooled to 4 ° C. A catalytic amount of 10% palladium-carbon was added with stirring. $H_2$ gas was then bubbled through the stirred reaction mixture at 4 ° C. After 10 min the solution was warmed to 25 ° C and hydrogen addition was continued for an additional 12 hrs. The solution was then filtered to remove the catalyst. The product tested positive for primary amine by a fluorescamine assay.

The above solution was stirred at 25 ° C with the slow addition of 0.1 ml thiophosgene (5 hrs). The solution was then evaporated to an orange sticky film. IR (KBr pellet) showed NCS peak at 2106 $cm^{-1}$. The methyl esters were hydrolyzed and evaporated to dryness.

Synthesis of LiLo1:

LiLo-methyl ester (1X) was also obtained by a mixed anhydride method.

1.0 g (0.0025 M) DTPA was suspended in 30 ml $CH_2Cl_2$ with 2 ml triethylamine under $N_2$ ATM. The solution was stirred for 24 hours at 25°C., then cooled to approximately-10°C. in an ice/salt bath. 250 ml (0.0025 M) ethyl chloroformate was added to the solution dropwise. The solution was stirred for 25 min., 367 mg VIII (0.00125 M) was dissolved in 5 ml $CH_2Cl_2$ with 0.5 ml triethylamine, and cooled to -5°C. This was added to the mixed anhydride solution dropwise with strong stirring. The solution was kept below 0°C. for 2 hours, then slowly allowed to warm to room temperature and let stir overnight. The solution was then evaporated and the residue dissolved in dry $CH_3OH$ (50 ml), 4 ml thionyl chloride was added dropwise under $N_2$ ATM. The solution was then heated to reflux for 5 hours. The volatiles were evaporated and the residue run down a silica column ($CH_2Cl_2$ with $CH_3OH$ gradient). IR (KBr Pellet) 1742 $cm^{-1}$, 1664 $cm^{-1}$, 1520 $cm^{-1}$, 1347 $cm^{-1}$, 1207 $cm^{-1}$.

EXAMPLE IV

LiLo1 was also be prepared by an alternate method using DTPA dianhydride. 20mg (VIII) was dissolved in 10ml water. 105 mg DTPA dianhydride was added and the solution shaken until dissolved. This was maintained at 4°C. for 5 days, then reduced immediately. A catalytic amount of 10% Pd/C was added and hydrogen was bubbled through the solution for 6 hrs. at room temperature, then stirred under a hydrogen atmosphere overnight. The solution was filtered, converted to isothiocyante using thiophosgene, and the excess thiophosgene extracted with ether. IR spectrum showed a peak at 2126 $cm^{-1}$, characteristic of isothiocyanate.

## Claims

**1.** A bifunctional chelating agent of the structure

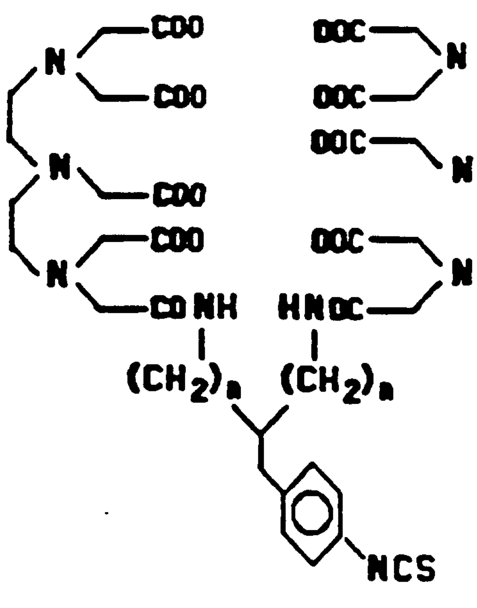

wherein n is 1 or 2.

**2.** A complex comprising a radionuclide bound to the chelating agent of claim 1.

**3.** A complex comprising a metal bound to the chelating agent of claim 1.

**4.** A complex comprising a polypeptide bound to the chelating agent of claim 1.

**5.** The complex of claim 4, wherein the polypeptide is an antibody.

6. The complex of claim 4, comprising a radionuclide bound to the chelating agent.

**Patentansprüche**

1. Bifunktioneller Chelatbildner mit der Struktur

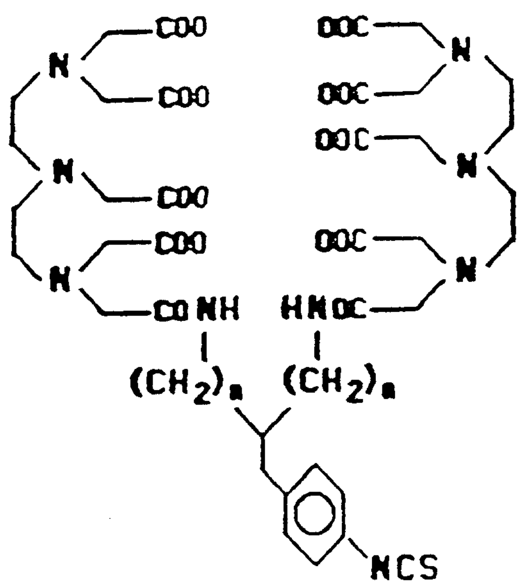

worin n 1 oder 2 ist.

2. Komplex, umfassend ein Radionuklid, das an den Chelatbildner von Anspruch 1 gebunden ist.

3. Komplex, umfassend ein Metall, das an den Chelatbildner von Anspruch 1 gebunden ist.

4. Komplex, umfassend ein Polypeptid, das an den Chelatbildner von Anspruch 1 gebunden ist.

5. Komplex nach Anspruch 4, worin das Polypeptid ein Antikörper ist.

6. Komplex nach Anspruch 4, umfassend ein Radionuklid, das an den Chelatbildner gebunden ist.

**Revendications**

1. Un agent chélatant bifonctionnel de structure:

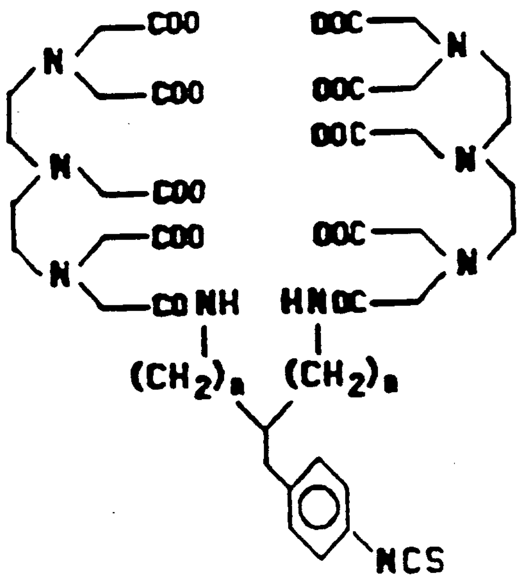

dans laquelle n est égal à 1 ou 2.

2. Un complexe comprenant un radionucléide lié à l'agent chélatant selon la revendication 1.

3. Un complexe comprenant un métal lié à l'agent chélatant selon la revendication 1.

4. Un complexe comprenant un polypeptide lié à l'agent chélatant selon la revendication 1.

5. Le complexe selon la revendication 4, dans lequel le polypeptide est un anticorps.

6. Le complexe selon la revendication 4, comprenant un radionucléide lié à l'agent chélatant.

FIGURE 1

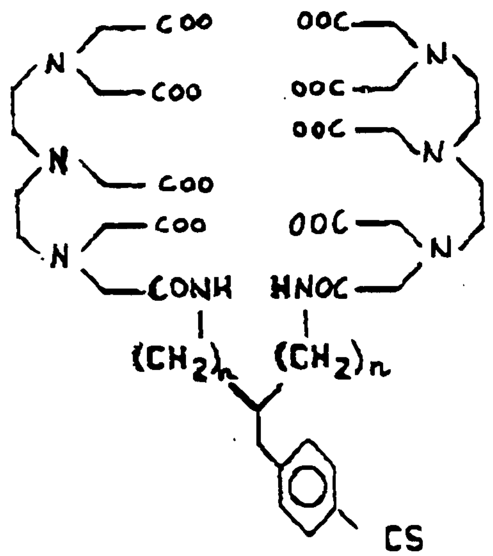

n = 1, "a" LiLo1

n = 2, "b" LiLo2

FIGURE 2A

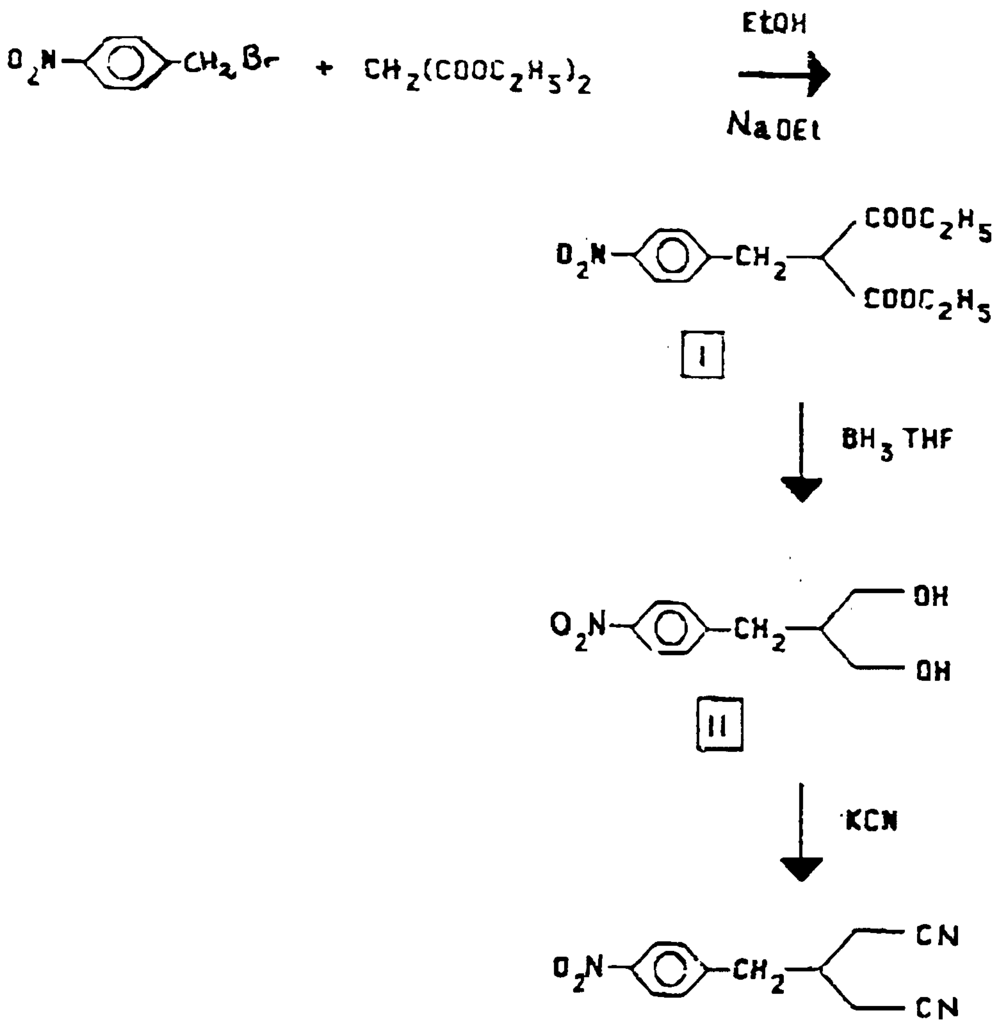

FIGURE 2B

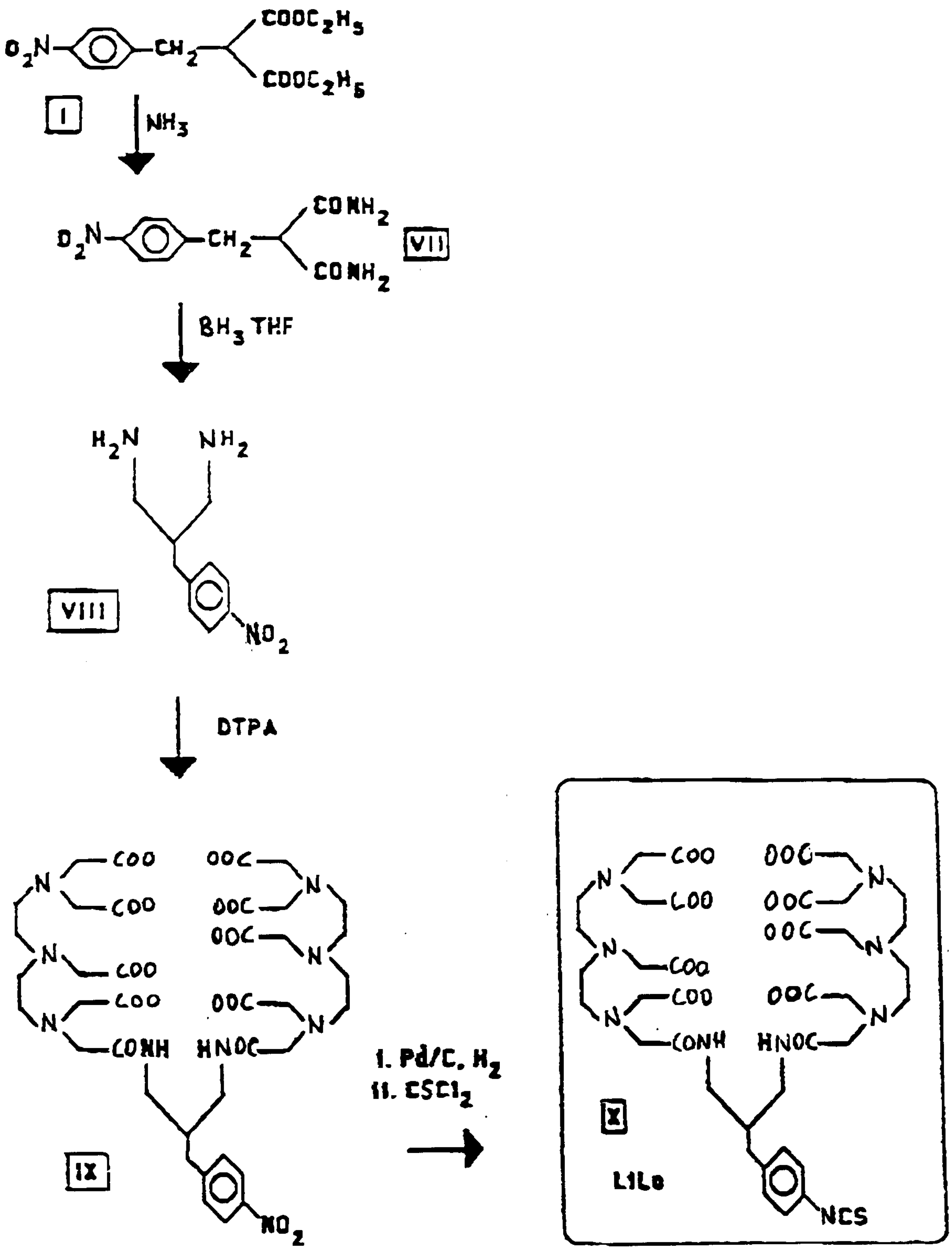

FIGURE 3

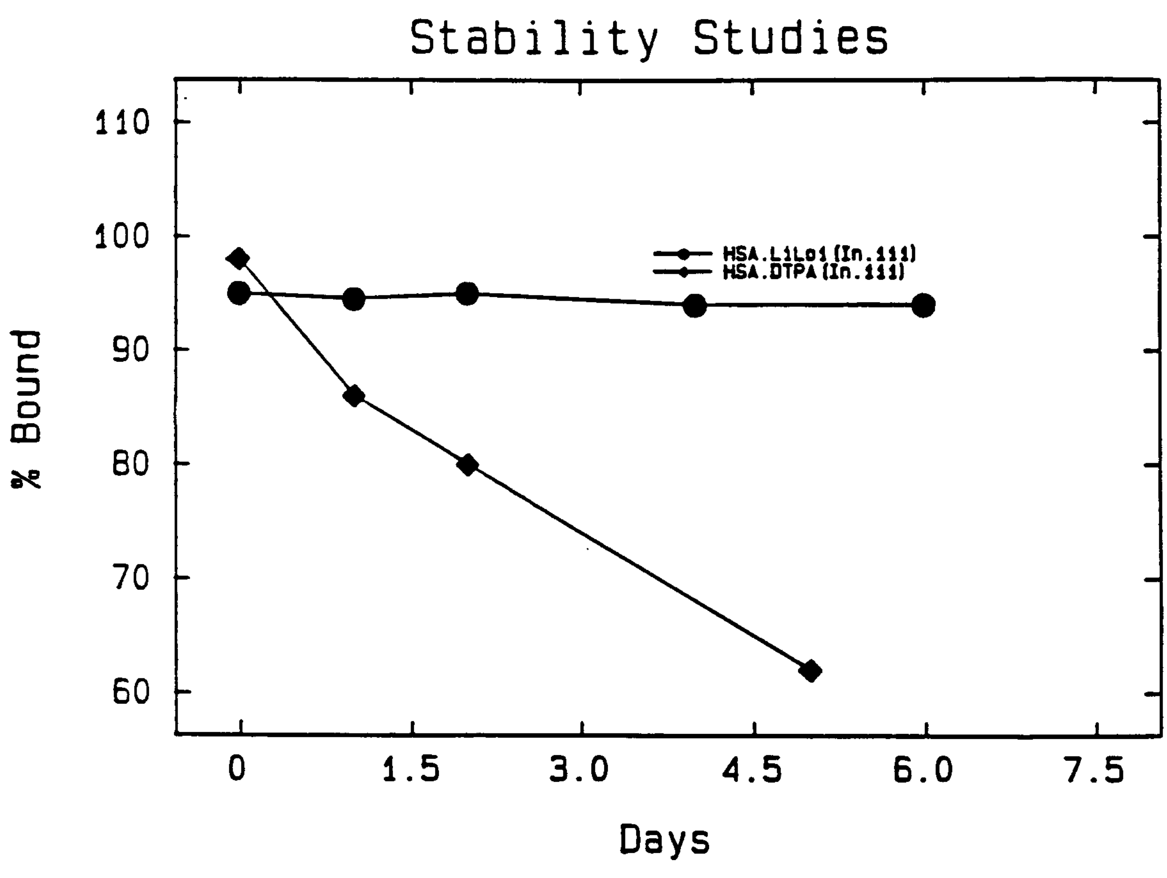
Stability Studies
110
100
90
80
70
60
% Bound
0
1.5
3.0
4.5
6.0
7.5
Days
HSA.L1Lo1 (In.111)
HSA.DTPA (In.111)

FIGURE 4

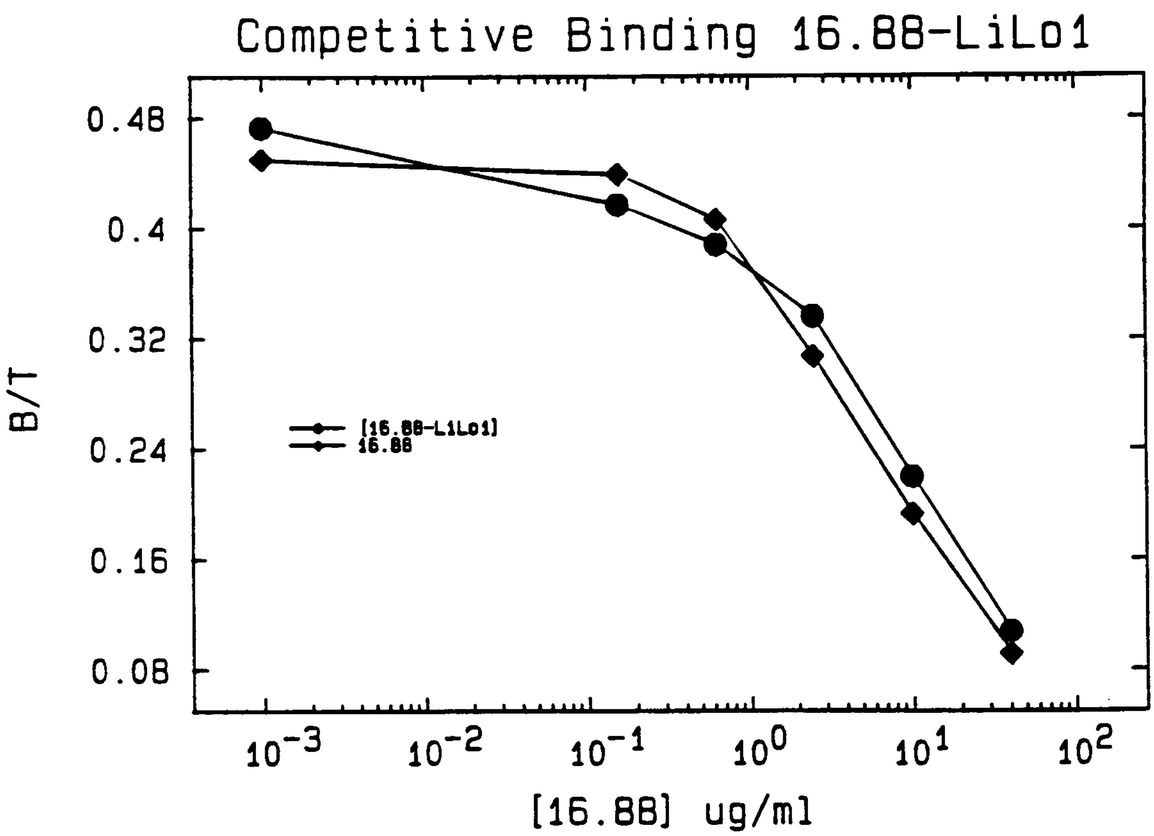
Competitive Binding 16.88-LiLo1
0.48
0.4
0.32
0.24
0.16
0.08
B/T
[16.88-LiLo1]
16.88
$10^{-3}$
$10^{-2}$
$10^{-1}$
$10^{0}$
$10^{1}$
$10^{2}$
[16.88] ug/ml

FIGURE 5

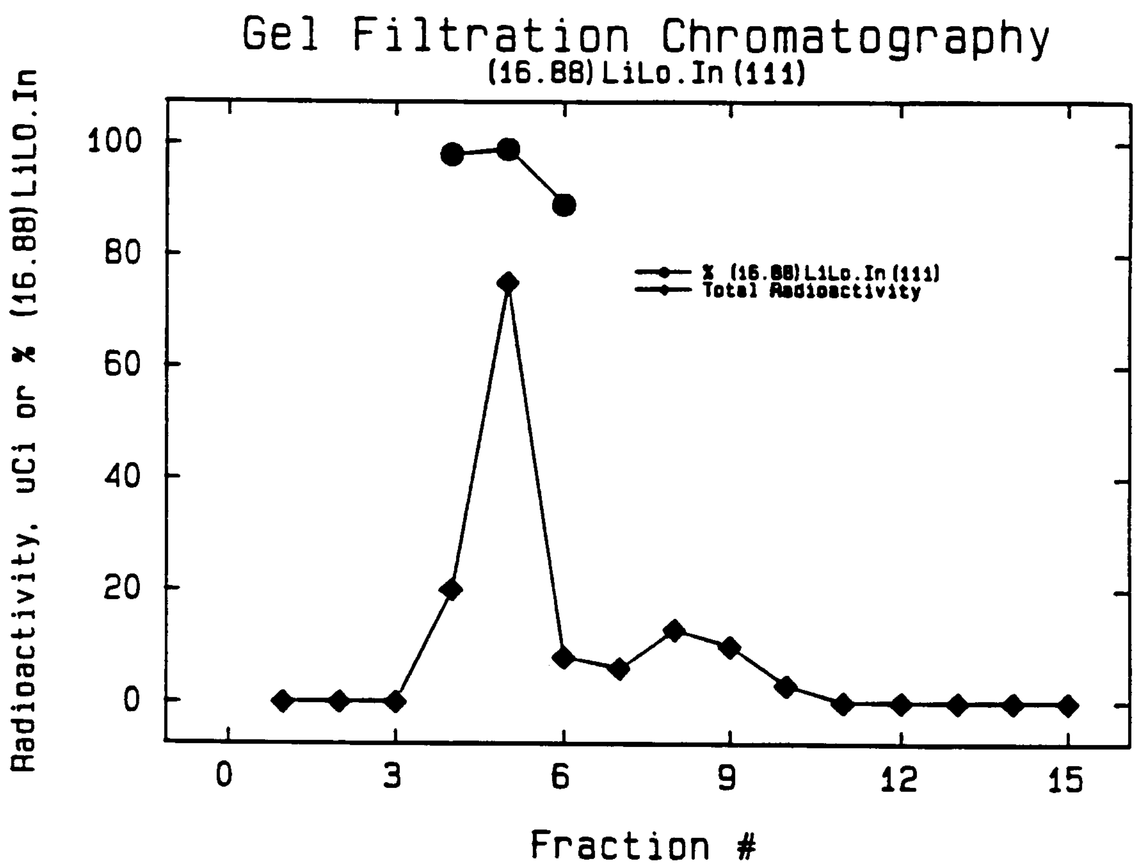
Gel Filtration Chromatography
(16.88) LiLo.In (111)
Radioactivity, uCi or % (16.88) LiLO.In
100
80
60
40
20
0
% (16.88) LiLo.In (111)
Total Radioactivity
0
3
6
9
12
15
Fraction #